Europäisches Patentamt

European Patent Office

Office européen des brevets

(19)

(11) Veröffentlichungsnummer: **0 515 003 A1**

## (12) EUROPÄISCHE PATENTANMELDUNG

(21) Anmeldenummer: **92201843.7**

(22) Anmeldetag: **20.04.90**

(51) Int. Cl.5: **A61F 2/36**

This application was filed on 23 - 06 - 1992 as a divisional application to the application mentioned under INID code 60.

(30) Priorität: **25.04.89 SE 8901509**
**13.09.89 US 406587**

(43) Veröffentlichungstag der Anmeldung:
**25.11.92 Patentblatt 92/48**

(60) Veröffentlichungsnummer der früheren Anmeldung nach Art. 76 EPÜ: **0 396 520**

(84) Benannte Vertragsstaaten:
**AT BE CH DE DK ES FR GB GR IT LI LU NL SE**

(71) Anmelder: **Branemark, Per-Ingvar**
**Ändergatan 3**
**S-431 39 Mölndal(SE)**

(72) Erfinder: **Branemark, Per-Ingvar**
**Ändergatan 3**
**S-431 39 Mölndal(SE)**

(74) Vertreter: **Barnieske, Hans Wolfgang**
**c/o H.W. Barnieske Patentbyra AB P.O. Box**
**25 Turingegatan 26**
**S-151 21 Södertälje 1(SE)**

(54) **Verankerungselement zum Halten eines Gelenkteils eines Hüftgelenks.**

(57) Das Verankerungselement (6") besitzt eine zumindest teilweise hohle Führungs- und Zentrierbuchse (8) zum Halten des Gelenkteils. Das Verankerungselement verträgt sich mit dem Knochen- und Knochenmarkgewebe und hat eine Oberfläche, welche wenigstens teilweise mit dem Gewebe verknöchernd zusammenwachsen kann, um eine dauerhafte Verankerung in dem Knochen- und Knochenmarkgewebe zu erreichen. Das Verankerungselement kann zur Wiederherstellung eines Hüftgelenks verwendet werden.

Fig.1

Rank Xerox (UK) Business Services

EP 0 515 003 A1

Die vorliegende Erfindung betrifft ein Verankerungselement zum Halten eines Gelenkteils eines künstlichen Hüftgelenks und ein künstliches Hüftgelenk.

## Beschreibung verwandter Gebiete

Über eine beträchtliche Zeitspanne wurden Versuche unternommen, zerstörte Gelenke durch künstliche Prothesen zu ersetzen. Diese sind in dem Knochengewebe entweder direkt oder mittels einer zwischenliegenden, adhäsiven Schicht, üblicherweise Knochenzement, verankert worden. Für die Prothesen wurden verschiedene Materialien, wie rostfreier Stahl, Chrom- und Kobaltlegierungen, Titanlegierungen, Aluminiumlegierungen, keramische Werkstoffe, Kohlefasern und viele andere Materialien verwendet. Man hat eine große Erfindungsgabe auf die Oberfläche und die Oberflächenbeschaffenheiten aufgewendet, von denen man annahm, daß man die bestmögliche Stabilität der Verankerung erreicht.

Das größte Problem der orthopädischen Chirurgie mit Prothesen ist nach wie vor, wie gut die Komponenten der Prothese im Knochengewebe des Patienten gesichert sind. Experimente, die eine feste mechanische Verbindung zwischen Implantat und Knochengewebe mit sich bringen ("Preß-Fit", vgl. EP 149 527), und die Verwendung von Knochenzement (Polymethylmetacrylat) haben nicht zu zufriedenstellenden Ergebnissen geführt.

Konventionellerweise werden die Prothesen in Knochenzement gesichert. Es wurde große Sorgfalt darauf verwendet, das Knochenmark von dem freigelegten Knochen zu entfernen - der Hohlraum ist sogar sauber ausgespült worden, bevor die Prothese eingesetzt und dann in dem Knochenzement gesichert wurde. Die einzementierten Prothesen lockern sich jedoch oft. Dies ist besonders bei Prothesen der Fall, die in ein lasttragendes Gelenk, wie Fußgelenke und Hüftgelenke, einzementiert sind. Es ist auch herausgefunden worden, daß Knochenzement, wenn er vor dem Hartwerden eingesetzt wird, dazu neigt, in das angrenzende Knochengewebe einzusickern. Davon ausgehend bietet dieses Einsickern eine gewisse mechanische Stabilität, aber bei jungen, übergewichtigen oder aktiveren Personen wird das Gelenk zwischen Knochen und Zement letztendlich aufgrund von Mikro-Verschiebungen zerstört. Es wächst eine Schicht eines Verbindungsgewebes zwischen Prothese und Knochen und schließlich lockert sich das Gelenk.

Ein anderes heute angewandtes Verfahren ist eine zementlose Methode, die auf eine biologische Sicherung der Prothese abzielt, z.B. wird ein direkter Kontakt zwischen Prothese und Knochengewebe ohne Zwischenschicht eines Verbindungsgewebes oder einer adhäsiv wirkenden Schicht gewünscht. Um eine zufriedenstellende Stabilität der Verankerung zu erreichen, sind verschiedene Materialien und Oberflächenstrukturen für die Prothese ausgetestet worden und es wurde eine Technik angewendet, die als "Preß-Fit" bekannt ist, um die Prothese nach dem Einsetzen in ihrer Position festzuhalten. Kürzlich veröffentliche Experimente (vgl. Schimmel et Huiskes, "Primary fit of the Lord cementless total hip", Acta Orthop. Scand. 1988: 59-(6): 638-642 lassen jedoch erkennen, daß diese Methode in Wirklichkeit nicht funktioniert. Es hat sich gezeigt, daß diese Methode zu einer extrem ungünstigen Drei-Punkte-Belastung mit Spannungskonzentrationen führt (EP 176 711).

Das Problem kann nicht bloß dadurch gelöst werden, daß eine "geeignete Oberfläche eines Materials" ausgewählt wird. Es gibt eine Reihe anderer Faktoren, die von entscheidender Bedeutung sind.

Dazu werden in den vergangenen Jahren Versuche unternommen, Halterungen aus Titan in die Knochenmarkshöhlung des Knochens zu verankern, um verknöchernd einzuwachsen, wie es von Hagert et al. "Metalcarpophalangal Joint Replacement with Osseo-integrated Endoprostheses" in Scand. J. Plast. Reconstr. Surg 20: Seiten 207-218, 1986, beschrieben ist.

Es ist bekannt orale und extra-orale Prothesen im Knochengewebe zu verankern. Diese Technik des verknöchernden Zusammanwachsens in der Zahnmedizin wurde von Prof. Brånemark und seinen Kollegen in den letzten 25 Jahren mit ausgezeichneten Ergebnissen entwickelt, indem Halterungen in den Backenknochen zum Halten von Zähnern und Brücken eingesetzt werden. Die Experimente jedoch, die von Hagert durchgeführt wurden, um diese Technik auf die Wiederherstellung von Fingergelenken zu übertragen, haben die Erwartungen nicht erfüllt. Die unakzeptabelen Ergebnisse sind offenbar auf die vollkommen unterschiedlichen Bedingungen zurückzuführen, die bei der Verwendung der "Technik der Zahnmedizin" bei der prothetischen Wiederherstellung von Fingergelenken auftreten. Diese Probleme sind selbstverständlich im Fall von Fußgelenk- und Hüftgelenk-Prothesen noch erschwert, nachdem vollkommen verschiedene Lasten auf diese Gelenke wirken.

Heute besteht das hauptsächliche Problem in der orthopädischen Chirugie mit Prothesen nach wie vor darin, daß sich die Knochenverankerungseinheit lockert. Jedoch treten mit einer Erfolgsrate von mehr als 90 % über einen Zeitraum von 20 Jahren eine Reihe anderer Probleme auf, die insoweit bisher nicht in Betracht gezogen werden mußten. Eines der hauptsächlichen Probleme ist eine zunehmende Abnutzung des Gelenkteils. Es ist eine andere Art der Ausführung von Prothesen im Vergleich zur bisher gebräuchlichen gefragt, wenn das verknöchernd zusammenwachsende Verfahren angewendet werden soll. Um das Gelenkteil austauschen zu können, ohne die Knochenverankerung zu beschädigen, muß das System der Prothese in Komponenten aufgeteilt werden, bei denen das Gelenkteil von dem tatsächlichen Verankerungselement abgetrennt werden kann. Darüber hinaus, wenn das zweistufige Verfahren angewendet wird, muß es möglich sein, das Gelenkteil in einem zweiten Schritt zu verbinden, wenn der Patient, oder zumindest das künstliche Gelenk des Patienten, nicht unbeweglich werden soll. Dazu müssen zwei Umstände in Betracht gezogen werden: Erstens, das Gelenkteil unterliegt der Abnutzung und muß deshalb austauschbar sein. Zweitens muß das Gelenkteil austauschbar sein um das zweistufige Verfahren anwenden zu können.

## Zusammenfassung der Erfindung

Es ist nun überraschenderweise festgestellt worden, dass die Probleme und Nachteile der oben beschriebenen Techniken durch die vorliegende Erfindung eliminiert werden können.

Die Erfindung betrifft ein Verankerungselement zum Halten eines Gelenkteils eines künstlichen Hüftgelenks, wobei das Verankerungselement eine zumindest teilweise hohle Führungs- und Zentrierbuchse zum Halten des Gelenkteils aufweist, wobei das Verankerungselement aus einem Material geformt ist, das sich mit dem Knochen- und Knochenmarkgewebe verträgt, wobei das Verankerungselement eine Oberfläche hat, die zumindest teilweise verknöchernd mit dem Gewebe zusammenwachsen kann, um eine dauerhafte Verankerung in dem Knochen und Knochenmarkgewebe zu erreichen.

Die Erfindung betrifft auch ein künstliches Hüftgelenk.

Andere Eigenschaften und Vorteile der Erfindung werden aus der folgenden Beschreibung der bevorzugten Ausführung der Erfindung mit dem Hinweis auf die begleitende Zeichnung deutlich, die in perspektivischer Ansicht die Wiederherstellung eines Hüftgelenks unter Verwendung eines Verankerungselements zeigt.

## Beschreibung der bevorzugten Ausführung

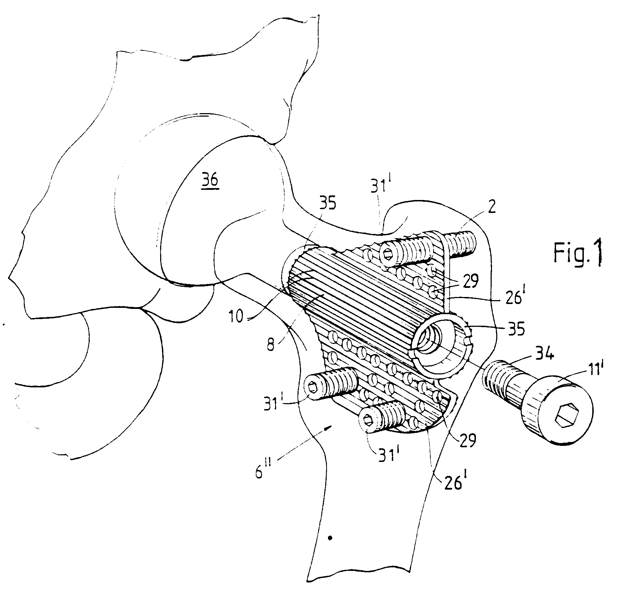

Fig. 1 stellt ein künstliches Hüftgelenk dar, unter Verwendung eines Verankerungselements 6'', das eine zentrale Führungsbüchse 8 mit längsverlaufender Riffelung 10, die in Einführungsrichtung ausgerichtet ist, aufweist. Das Verankerungselement 6'' ist aus Platzgründen mit zwei flügelartigen Platten 26' versehen, die aus der Buchse 8 herausragen. Die Platten 26' haben Durchgangslöcher 29 mit Schneidkanten.

In den Platten 26 sind auch Durchgangslöcher für Schrauben 31 vorgesehen. Die Schrauben 31' haben unterschiedliche Durchmesser, so daß, wenn sie in die Löcher eingeschraubt werden, sie in Verbindung mit der Platte 26' treten und folglich verhindert wird, daß die Enden 2 in dem Knochen Schaden verursachen. Die Platten 26' sind auch mit Rillen 10 versehen, welche in Einführungsrichtung verlaufen, um ein günstiges Einwachsen der Verankerungselemente 6'' in das Knochen- und Knochenmarksgewebe zu ermöglichen. Das Ende der Fünrungsbuchse 8, das dem Hüftgelenk abgekehrt ist, ist mit einer zapfenähnlichen Schraube 11 verschlossen. Das untere, dem Gelenk zugekehrte Ende der Schraube ist mit einem Gewinde 34 versehen, so daß die Schraube 11' in das Ende 35 der Buchse 8, die ein künstliches Gelenk 36 aufnimmt, eingeschraubt werden kann. Alle Teile des Verankerungselementes 6'' sind aus Titan gemacht.

Bei einer Operation wird ein seitlicher Einschnitt gemacht und es wird durch die seitlichen Schichten bis in das Collum hinein ein Kürschnerdraht eingeführt. Gleichzeitig ermöglicht eine Durchleuchtung eine Orientierung in zwei Ebenen. Dann wird ein relativ großes Loch zur Aufnahme der Buchse 8 gebohrt, und es werden Schlitze zur Aufnahme der Platten 26' geformt. Dann werden senkrecht dazu Löcher für die Schrauben 31' gebohrt. Die letzteren Löcher haben zwei verschiedene Durchmesser, entsprechend der Durchmesser der Schrauben 31'. Dann wird dem Gelenk ermöglicht, einzuwachsen. Schließlich wird das künstliche Gelenkteil 36 im Anschluß an den Zeitraum zum Einwachsen angebracht. Alternativ dazu kann die gesamte Operation in einem Schritt erfolgen.

Die Verankerungselemente wirken als Vorbereitungsinstrument, wenn sie in dem Gewebe eingesetzt sind. Alternativ dazu können spezielle Instrumente zur Vorbereitung benutzt werden, um optimale Bedingungen zum Einwachsen in den Knochen zu erreichen und können als zusätzliche Halterung und weiterhin zur Optimierung der Stabilität dort belassen werden. Das Einwachsen hängt teilweise von den Schneidkanten der Verankerungselemente ab, die eine perfekte Übereinstimmung mit dem umgebenden Gewebe ergeben.

Das Grundprinzip der vorliegenden Erfindung ist folglich die Erkenntnis, daß Knochen- und Knochenmarksgewebe eine strukturelle und funktionelle Einheit bilden, und die Wechselwirkung zwischen ihnen diesbezüglich berücksichtigt werden muß, wenn ein chirurgischer Austausch vorgenommen werden soll. Das bedeutet, daß soweit wie technisch möglich, ein Verbindungsweg zwischen Knochenmark und Knochengewebe ermöglicht werden muß.

Die oben erwähnten Verankerungsschrauben können wie die Verankerungselemente, die in meiner anhängigen US-Anmeldung US 406 486 gezeigt wird, geformt sein, welche als ein hohler, im wesentlichen zylinderbuchsenartiger Körper mit einem Außengewinde und, falls zweckmässig, auch mit abnehmender Wanddicke, ausgebildet ist. Das offene Ende des Körpers kann konisch verjüngt sein und kann mit Schlitzen, die am offenen Ende anfangen, versehen sein. Es wird somit ein selbständiges Gewindeschneiden erreicht, wenn der schraubenartige Körper in den Knochen eingeschraubt wird und das entfernte Knochengewebe innerhalb der Schraube aufgenommen wird um ein geeignetes, verknöcherndes Einwachsen sicherzustellen.

Obwohl die Erfindung in Verbindung mit einer bevorzugten Ausführungsart beschrieben worden ist, werden dem Fachmann viele andere Abwandlungen und Modifikationen und andere Einsatzmöglichkeiten eröffnet. Es wird deshalb vorgebracht, daß die Erfindung nicht durch diese spezielle Darlegung beschränkt ist, sondern nur durch die anhängenden Ansprüche.

**Patentansprüche**

1. Verankerungselement (6'') zum Halten eines Gelenkteils eines künstlichen Gelenks, insbesondere eines Hüftgelenks, wobei das das Verankerungselement (6'') eine zumindest teilweise hohle Führungs- und Zentrierbuchse (8) zum Halten des Gelenkteils aufweist, wobei das Verankerungselement (6'') aus einem Material geformt ist, das sich mit Knochen- und Knochenmarkgewebe verträgt, wobei das Verankerungselement (6'') eine Oberfläche hat, die zumindest teilweise verknöchernd mit dem Gewebe zusammenwachsen kann, um eine dauerhafte Verankerung in dem Knochen und Knochenmarkgewebe zu erreichen, dadurch **gekennzeichnet**, dass
   a) die Führungs- und Zentrierbuchse (8) eine längsverlaufende Riffelung hat (10), um das Einwachsen zu fördern,
   b) das Verankerungselement (6'') zusätzlich wenigstens eine wesentlich, flache flügelartige Platte (26') aufweist, die aus den Führungs- und Zentrierbüchse (8) herausragt und ein an einem der Führungs- und Zentrierbüchse abgewandten Ende vorgesehenes Durchgangsloch zur Aufnahme einer Verankerungsschraube (31') hat.

2. Verankerungselement nach Anspruch 1, dadurch **gekennzeichnet**, dass die flügelartige Platte (26') eine längsverlaufende Riffelung und Durchgangslöcher mit Schneidkanten (29) aufweist.

3. Verankerungselement nach Anspruch 1 oder 2, dadurch **gekennzeichnet**, dass die im Durchgangsloch vorgesehene Schraube (31') zwei verschiedene Durchmesser zur ihrer Positionierung (31') bezüglich der flügelartigen Platte (26') hat.

4. Verankerungselement nach einem der Ansprüche 1 bis 3, dadurch **gekennzeichnet**, dass das Verankerungselement (6'') gänzlich aus Titan besteht.

5. Künstliches Hüftgelenk, dadurch **gekennzeichnet**, dass es ein Gelenkteil aufweist, das von einer wenigstens teilweise hohlen Führungs- und Zentrierbuchse (8) eines Verankerungselements (6, 6') nach einem der Ansprüche 1 bis 4 gehalten wird.

Fig.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | WO-A-8 801 854 (JOINT MEDICAL PRODUCTS CORP.) <br> * Seite 7, Zeile 8 - Zeile 16; Abbildungen 1,3 * <br> * Seite 14, Zeile 6 - Zeile 12 * <br> --- | 1,4,5 | A61F2/36 |
| A | FR-A-989 341 (STEENBRUGGHE) <br> * Abbildungen 2-5 * <br> --- | 1,5 | |
| A | DE-A-3 006 179 (INSTITUT STRAUMANN AG) <br> * Seite 11, Absatz 2; Anspruch 1; Abbildungen * <br> --- | 1,4,5 | |
| A | DE-A-2 808 740 (OMAR - PACHA) <br> * Ansprüche 1,3; Abbildungen 1-3,6 * <br> --- | 1,5 | |
| A | EP-A-0 099 167 (CARBOMEDICS INC.) <br> * Seite 6, Zeile 7 - Zeile 15; Anspruch 1; Abbildungen 1,6,7 * <br> --- | 1,5 | |
| A | US-A-4 488 319 (VON RECUM) <br> * Zusammenfassung * | 1,5 | |
| | ----- | | **RECHERCHIERTE SACHGEBIETE (Int. Cl.5)** <br><br> A61F |

Der vorliegende Recherchenbericht wurde für alle Patentansprüche erstellt

| Recherchenort | Abschlußdatum der Recherche | Prüfer |
|---|---|---|
| BERLIN | 13 AUGUST 1992 | KANAL P. |